(19) **Europäisches Patentamt**

**European Patent Office**

**Office européen des brevets**

(11) **EP 1 154 953 B1**

(12) **EUROPÄISCHE PATENTSCHRIFT**

(45) Veröffentlichungstag und Bekanntmachung des Hinweises auf die Patenterteilung:
**16.11.2005 Patentblatt 2005/46**

(21) Anmeldenummer: **00907582.1**

(22) Anmeldetag: **18.02.2000**

(51) Int Cl.$^7$: **C01B 15/055**

(86) Internationale Anmeldenummer:
**PCT/EP2000/001350**

(87) Internationale Veröffentlichungsnummer:
**WO 2000/048940 (24.08.2000 Gazette 2000/34)**

(54) **PEROXOCHLORSAURE, DERIVATE UND ANIONEN, SALZE DAVON, UND VERFAHREN ZU DEREN HERSTELLUNG UND VERWENDUNG**

PEROXOCHLORIC ACID, DERIVATIVES AND ANIONS, SALTS THEREOF, METHOD FOR PRODUCING THEM AND USE OF THE SAME

ACIDE PEROXOCHLORIQUE, SES DERIVES ET ANIONS, LEURS SELS, LEUR PROCEDE DE PRODUCTION ET LEUR UTILISATION

(84) Benannte Vertragsstaaten:
**AT BE CH CY DE DK ES FI FR GB GR IE IT LI LU MC NL PT SE**

(30) Priorität: **19.02.1999 DE 19907256**
**20.10.1999 DE 19950632**

(43) Veröffentlichungstag der Anmeldung:
**21.11.2001 Patentblatt 2001/47**

(73) Patentinhaber:
• **Stahl, Kurt-Wilhelm, Prof. Dr. Dr.**
**79100 Freiburg (DE)**
• **CytoTools GmbH**
**64287 Darmstadt (DE)**

(72) Erfinder: **Stahl, Kurt-Wilhelm, Prof. Dr. Dr.**
**79100 Freiburg (DE)**

(74) Vertreter: **Gille Hrabal Struck Neidlein Prop Roos Patentanwälte**
**Brucknerstrasse 20**
**40593 Düsseldorf (DE)**

(56) Entgegenhaltungen:
**EP-A- 0 093 875          WO-A-84/03274**
**DE-B- 1 567 465          DE-B- 1 567 816**

• **YONGHAO NI: "Mechanism and Kinetics of Chlorine Dioxide Reaction with Hydrogen Peroxide Under Acidic Condition" THE CANADIAN JOURNAL OF CHEMICAL ENGINEERING, Bd. 75, Februar 1996 (1996-02), Seiten 31-36, XP000864568**

**Beschreibung**

Die Erfindung betrifft Chlorhydroperoxid, Peroxochlorsäure, deren Desoxo-Dimere, und/oder Kohlendioxidaddukte, und/oder Anionen und/oder Salze dieser Verbindungen. Sie betrifft auch die Herstellung dieser Verbindungen und deren Verwendung im pharmazeutischen und insbesondere im medizinischen Bereich als Arzneimittel und Desinfektionsmittel, wie in der Kosmetik und der medizinischen Pflege als gewebeverträgliches Desodorans, sowie in den Bereichen Nahrungsmittelbehandlung und Technologie, insbesondere bei der Konservierung von Nahrungs- und Genußmitteln und als Bleichmittel sowie zur Trinkwasserdesinfektion, Entkeimung von Pflanzen und Früchten in der Landwirtschaft und als Oxidationsmittel in der technischen Chemie sowie zur Abgasreinigung.

<u>Hintergrund der Erfindung</u>

Oxidationsmittel finden vielfältigste Anwendungen in der technischen Chemie, in der Hygiene und Nahrungsmittelkonservierung, in der Kosmetik und auch in der Pharmazie.

Nach. Polly Matzinger (Polly Matzinger: "Tolerance, Danger, and the Extended Family" in Annu. Rev. Imniunol. 1994,12) senden gewaltsam, d.h. durch massive Strahleneinwirkung, toxische Substanzen, parasitäre, bakterielle oder virale Infektionserreger, lytisch, nicht-apoptotisch, sterbende Zellen Gefahrensignale ("danger signals") aus, welche andauern müssen, damit die körpereigene Abwehrkraft, die neben dem eigentlichen Antigensignal einer unspezifischen Costimulation durch Antigenpräsentierende Zellen (z.B. Makrophagen) bedarf, klinisch optimal wirksam werden kann.

Bei einem gewaltsamen, nicht-apoptotischen Zelluntergang sind Freßzellen (sogenannte Mikro- und Makrophagen) für die Zelltrümmerbeseitigung verantwortlich. Bei dieser Zelltrümmerbeseitigung werden oxidativ wirksame Sauerstoffmetabolite freigesetzt. Wasserstoffperoxid ($H_2O_2$) ist der bekannteste Vertreter. In-vitro-Versuche zeigen, daß $H_2O_2$ im mikromolaren Bereich über Aktivierung des Transkriptionsfaktors. NF-κB zu einer Immunmodulation von Lymphozyten führen kann (R. Schreck et al., The EMBO Journal <u>10</u>(8), 2247-58 (1991); M. Los et al., Eur. J. Immunol. <u>25</u>, 159-65 (1995)). Der Arbeitskreis von Avraham Novogrodsky war der erste, der in vitro gezeigt hat, daß bestimmte Oxidantien (Bowers W.E.: "Stimulation of Lymphocytes with periodate or Neuraminidase plus Galactose Oxidase -'NAGO" p: 105 - 109, Review in immunochemical Techniques Part K Methods in Enzymology Vol. 150,1987) unter anderem auch das im Körper selber gebildete $H_2O_2$, co-mitogen die durch Antigenreiz hervorgerufene Lymphozytenvermehrung steigem, wenn sich gleichzeitig Makrophagen in der Lymphozytenkultur befinden (Stenzel K.H., Rubin A.L., Novogrodsky A.: "Mitogenic and Comitogenic Properties of Hemin." J.Immunol. 127, 6: 2469-2473 et ibid. cit. ref.). Werden die oxidativ wirksamen Sauerstoffmetabolite im Körper in nicht ausreichendem Maße gebildet, bleibt eine immunabwehr unvollkommen bzw. sie bleibt sogar ganz aus, so daß eine Toleranz oder pathologische Anergie entsteht Werden sie überschießend bzw. schwelend, übermäßig langanhaltend produziert, dann entstehen chronische Entzündungen und Gewebsvernarbungen.

Es ist nach diesen Befunden von Avraham Novogrodsky im Zusammenhang mit der Hypothese von Polly Matzinger davon auszugehen, daß oxidativ wirksame Sauerstoffverbindungen besonders in solchen klinischen Situationen eine therapeutische Wirkung haben, in denen ihre körpereigene Bildung mangelhaft bleibt bzw. nachläßt, bevor die Körperschäden vollkommen wiederhergestellt und die Infektionserreger vollständig beseitigt wurden. Es ist besonders in den Fällen mit einem Behandlungserfolg zu rechnen, in denen die Erreger die Zellen zwar infizieren, sie aber nicht zerstören, so daß die "danger signals" ausbleiben. Beispielhaft seien hier Infektionen mit Lepra- und Tuberkelbazillen und solche mit Herpes- und AIDS (HIV) -Viren genannt.

Über den erfolgreichen klinischen Einsatz von Kaliumbichromat zur Abheilung von jauchenden chronischen Wunden wurde bereits 1909 berichtet (Fenwick, J.: *"The Treatment of Cancer by the Use of Potassium Bichromate",* British Medical Journal, March 6th, 1909, 589-591).

Zahlreiche weitere, in der Zwischenzeit erschienene Veröffentlichungen zeigen, daß in physiologischer Weise im Körper gebildetes Wasserstoffperoxid wie auch das in vivo noch kurzlebigere Peroxonitrit, das aus dem ebenfalls physiologischen, Nitroxid und Wasserstoffperoxid entstehen kann, ebenfalls Wundheileffekte zeigen, an welchen eine positive Immunmodulation essentiell beteiligt ist. Beispielsweise beschreibt die EP-A-0 390 829 eine Methode zur Steigerung der syngenen intradermalen Zellvermehrung durch Wachstumsfaktoren beim Menschen mit Wasserstoffperoxidinjektionen. Eine solche comitogene Steigerung der Wachstumsfaktorwirkung von Interleukin-2 wurde 1987 auch für Periodat beschrieben (Wang J. et al., The American Journal of Medicine 1987, 83: 1016 - 1023).

Es hat sich gezeigt, daß (co)mitogene Oxidantien nicht tolerable Nebenwirkungen haben, wie z.B.: bei Bichromat: die inzwischen bekannt gewordene krebserzeugende Wirkung von Chromoxid. Bei Periodat: Iodüberempfindlichkeit und toxische Wirkung. Deswegen muß der klinische Einsatz umständlich als "adoptive transfer" stattfinden, d.h. die Blutzellen werden entnommen, in vitro behandelt und dann in vivo zurückgegeben, wie in der zuvor zitierten Arbeit von J. Wang et al. 1987 beschrieben. Bei NAGO: die Fremdproteinsensibilisierung. Bei $H_2O_2$: die Bildung toxi-

scher Sauerstoffradikale. Auch stehen ihrer Verwendung als Arzneimittel technische Probleme entgegen, z.B.: bei $H_2O_2$: geringe Haltbarkeit in verdünnter wäßriger Lösung; die Katalaseempfindlichkeit mit massiver Sauerstoffgasfreisetzung. Bei oxidierten Ubichinon-Derivaten: Galenische Probleme und eingeschränkte Bioverfügbarkeit.

Es war daher bisher nicht möglich, die experimentell nachgewiesene immunpharmakologische Wirkung (co) mitogener Oxidantien auf die Geweberegeneration/Wundheilung, auf die Infektabwehr bzw. auf die Steigerung der Immunantwort auf die klinische Praxis zu übertragen, in der neben einer örtlichen Anwendung auch eine systemische Anwendung meist in Form einer intravenösen Verabreichung wünschenswert ist.

In der Patentliteratur sind einige Chlor-Sauerstoff-Präparationen beschrieben, die insbesondere in solchen technischen Bereichen Verwendung finden, in denen sie als Oxidantien nicht nur in der Industrietechnik als Bleichmittel und Geruchsbeseitiger eingesetzt werden, sondern auch zur paramedizinischen Verwendung empfohlen werden, wie in der Kosmetik zur Haut und Haarpflege, zur Haushaltpflege, im sanitären Sektor zur Hygiene und/oder als Desinfektionsmittel von Oberflächen (US 2,701,781; US 3,123,521) und/oder Wunden (US 4,084,747; EP-A-0 744 895), als Konservierungsmittel für Käse (US 3,147,124), zur Trink- und Badewasser-Aufbereitung (US 4,296,103; DE-A-44 05 800, DE-A-19 518 464; WO 96133947; WO 97/06098). Die US-A-4,296,103, EP-A-0 136 309, US-A-4,507,285 und EP-A-0 255145 beschreiben die Arzneimittelanwendung von Chlor-Sauerstoff-Präparationen. Jedoch erfüllen die beschriebenen Chlor-Sauerstoff-Präparationen die Anforderungskriterien einer modernen Arzneimittelzulassung nicht, die besagen, daß die Pharmakodynamik der Präparation einer chemisch definierten Verbindung als sogenannter Wirksubstanz zugeordnet werden können muß, auf die das Arzneimittelprodukt zu standardisieren ist, nicht zuletzt, um so eine gleichbleibende Arzneimittelqualität zu gewährleisten.

Aufgabe der Erfindung ist es daher, ein Oxidationsmittel bereitzustellen, das die vorstehenden Nachteile nicht aufweist. Das Oxidationsmittel soll neben der üblichen Anwendbarkeit für Oxidationszwecke auf technischem und medizinischem Gebiet, sowie zur Desinfektion, auch die Möglichkeit zur Formulierung als Arzneimittel zur örtlichen, wie auch systemischen Anwendung, z.B. zur intravenösen Verabreichung bieten, beispielsweise als Arzneimittel für die Geweberegeneration, die Wundheilung und zur Infektabwehr bzw. zur Steigerung der Immunantwort. Es soll darüber hinaus den Anforderungen der modernen Arzneimittelzulassung genügen.

Angaben zu den Zeichnungen

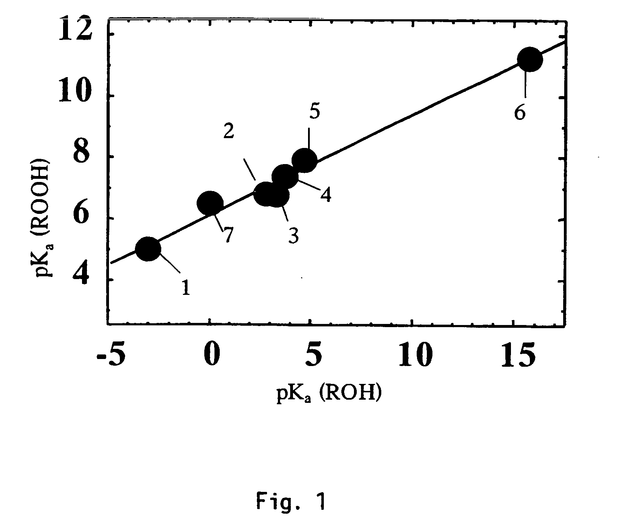

Fig. 1 zeigt die Korrelation zwischen dem pKa-Wert von Säuren (ROH; x-Achse) und entsprechenden Peroxidsäuren (ROOH; Y-Achse).
(1) = $HOONO_2/HONO_2$-Paar; (2) = $ClCH_2C(O)OOH/ClCH_2C(O)OH$-Paar; (3) = $HC(O)OOH/HC(O)OH$-Paar; (4) = $HOONO/HONO$-Paar; (5) = $CH_3C(O)OOH/CH_3C(O)OH$-Paar; und (6) $H_2O/H_2O$-Paar. (7) zeigt den Wert für das $O_2ClOOH$ (erfindungsgemässe Säure)/$O_2ClOH$-Paar.

Detaillierte Beschreibung der Erfindung

Überraschenderweise hat es sich gezeigt, daß diese Aufgabe gelöst werden kann durch die Bereitstellung von einem definierbaren Chlorhydroperoxid sowie von dessen Salzen und Anionen. Das erfindungsgemäß bereitgestellte Chlorhydroperoxid hat die Formel $HOOClO_2$, in der Chlor 5-wertig ist, und verhält sich als Säure, die in wäßrigem Milieu das Anion $^-OOClO_2$ liefert. Es wird daher als Peroxochlorsäure und sein Anion als Peroxochlorat bezeichnet.

Die Vereinigung zweier Peroxochlorationen kann unter Abspaltung eines Sauerstoffmoleküls zu Abkömmlingen (Derivaten) des Peroxochlorats führen mit einer Peroxogruppierung und zwei Chloratomen mit unterschiedlicher Wertigkeit. Diesen Ionen ist die Summenformel $Cl_2O_6^{2-}$ zuzuschreiben.

Chlorhydroperoxide stellen eine Verbindungsklasse dar, die bisher noch nie, weder als solche noch in einer ihrer anionischen Formen in Substanz oder auch in Lösung hergestellt und isoliert werden konnte. In der Fachliteratur sind von den Chlorhydroperoxiden bisher nur theoretische Stabilitäten von Molekülen für die Gasphase berechnet bzw. vorhergesagt worden. Die Berechnungen führen zum Teil zu widersprüchlichen Ergebnissen. Auch in der Gasphase der Atmosphäre konnte bisher kein Hinweis auf die Existenz von langlebigen Hydroperoxiden des Chlors gefunden werden (Finkbeiner, M., Crowley, J.N., Horie, O., Müller, R., Moortgat, G.K., und Crutzen, P.J., J. Phys. Chem. <u>99</u>, 16264-16275 (1995)).

In der Literatur wird berichtet, daß die Umsetzung von Chlordioxid mit Wasserstoffperoxid zu Chlorit und Sauerstoff führt (Bogdanchikov, G.A., Kozlov, Yu.N. and Berdnikov, V.M. *The Mechanism of the Elementary Act of* $HO_2$-*Anion Oxidation by a* $ClO_2$ *Radical in Aqueous Solution*. Khim. Fiz. <u>1983</u> (5), 628-636). Ni, Yonghao and Wang, Xiaolan, *(Mechanism and Kinetics of Chlorine Dioxide Reaction with Hydrogen Peroxide Under Acidic Conditions* Canadian J. of Chemical Engineering <u>75</u>, 31-36 (1996)) arbeiten bei einem pH-Wert von 3,63 bis 6,10 und erzielen Chlorit nach folgender Reaktionsgleichung

$$2\,ClO_2 + H_2O_2 + 2OH^- \Rightarrow 2\,ClO_2^- + O_2 + 2H_2O$$

Als unstabiles Zwischenprodukt postulieren sie eine Verbindung mit 4-wertigem Chlor.

Überraschenderweise hat es sich im Rahmen der vorliegenden Erfindung gezeigt, daß es möglich ist, stabile Peroxochlorsäure und stabile Salze bzw. Anionen davon herzustellen und zu isolieren. Es hat sich nämlich beispielsweise gezeigt, daß stabile Peroxochlorsäure bzw. deren Anionen und Salze erhalten werden durch Umsetzung von Chlordioxid mit Wasserstoffperoxid, wenn bei pH-Werten von gleich oder oberhalb des pKs-Wertes von Peroxochlorsäure (HOOClO_2) gearbeitet wird. Bevorzugt wird bei pH-Werten von $\geq 10$, besonders bevorzugt im pH-Bereich 10-12 gearbeitet.

Einen Gegenstand der Erfindung bildet daher Peroxochlorsäure oder deren Salze; sowie Peroxochlorsäure und deren Salze bzw. Anionen in wäßriger Lösung Die Erfindung umfaßt weiter Desoxo-drimere des Peroxochlorats mit gemischtvalenten Chloratomen und deren Salze bzw. Anionen in wäßriger Lösung sowie das Kohlendioxidaddukt, als Säure, als Anion in Lösung, oder als Salz davon. Die Desoxo-Dimere und/oder Kohlensäureaddukte werden im folgenden als "Derivate" bezeichnet, insbesondere $Cl_2O_6{}^{2-}$-Anionen, oder Salze davon, oder $O_2Cl$-O-O-C(=O)O$^-$-Anionen, oder Salze davon.

Die Peroxochlorsäure weist einen pKa-Wert (synonym pKs-Wert) von $6,5 \pm 0,2$ auf und unterscheidet sich so von den bekannten Chlor-Sauerstoff-Säuren, wie aus der nächstehenden Tabelle ersichtlich, in der auch weitere charakteristische Parameter der Säure bzw. von deren Anion im Vergleich mit solchen der bekannten Säuren angegeben sind.

Die Charakterisierung des Peroxochlorations gelingt neben dem pKa-Wert auch mittels einer charakteristischen Ramanbande bei 1051 cm$^{-1}$ (Tabelle 1)

Tabelle 1:

| Säure/Base-Paar | PKs-Wert der Säure | Bandenlage [cm$^{-1}$] im Raman-Spektrum des Säureanions | UV-Spektrum des Säureanions |
|---|---|---|---|
| HOCl/ $^-$OCl | 7,6 | 729 ± 1 (stark) | $\lambda_{max} = 298$ nm |
| HOClO/ $^-$OClO | 2,0 | 799 ± 1 (stark) | $\lambda_{max} = 260$ nm |
| HOClO$_2$/ $^-$OClO$_2$ | 0,0 | 933 ± 1 (stark) | Flanke$\lambda$<215 nm |
| HOClO$_3$/ $^-$OClO$_3$ | -10 | 937 ± 1 (stark) | Flanke$\lambda$<205 nm |

| HOOClO$_2$/ $^-$OOClO$_2$ | 6,5 ± 0,2 | 1051 ± 1 (stark), 983 ± 1 (schwach) | Flanke$\lambda$<230 nm |
|---|---|---|---|
| Carbonat, CO$_3^{2-}$ | | 1069 ± (stark) | |

Insbesondere lässt sich die Verbindung auch von anderen Oxoverbindungen und von Carbonat unterscheiden (Tabelle 2):

**Tabelle 2:**

| Verbindung | RAMAN-Linie (cm$^{-1}$) |
|---|---|
| Wasserstoffperoxid | 878 ± 1 (stark) |
| Peroxonitrit, ON-OO$^-$ | 1050 ± 1 (stark) |
| Peroxosalpetersäure. O$_2$N-OOH | 945 (O-O-Streckschwingung) |
| Peroxochlorat, O$_2$Cl-OO$^-$ | 1051 ± 1 (stark), 983 ± 1 (schwach) |
| Oxone®, $^-$O$_3$S-OOH | 1062 ± 1 (stark), 983 ± (schwach) |

Insbesondere die Ähnlichkeit mit der Peroxonitrit-Bande bei 1050 cm$^{-1}$ weist auf die strukturelle Verwandschaft hin.

Die Stabilität der Peroxochlorsäure in wäßriger Lösung kann durch die Halbwertzeit für ihren Zerfall bei Raumtemperatur angegeben werden. Sie liegt bei $t_{1/2} \approx$ 6 Minuten. In der Gasphase konnte zwischenzeitlich gezeigt werden, daß die Chlorperoxobindung dem Charakter nach zwischen einer van-der-Waals-Bindung und einer kovalenten Bindung liegt.

Das Peroxochloration an sich ergibt sich als stabile Verbindung. Da es jedoch in wäßriger Lösung im Gleichgewicht mit der Peroxochlorsäure steht, zerfällt es in Abhängigkeit vom pH-Wert. Die Halbwertzeit (in Tagen) für den Zerfall des Peroxochlorations läßt sich als Funktion der Protonenkonzentration mit der Gleichung berechnen:

$$t_{1/2} = 0,00412 + 3,85 \times 10^{-9} / [H^+] \text{ Tage} \qquad \text{(Gleichung 1)}$$

Bei pH 11 liegt sie bei etwa 400 Tagen; bei pH 12 bei etwa 10 Jahren.

Zur Verbesserung der Haltbarkeit der Peroxochlorate in wäßrigem Milieu ist es daher bevorzugt, die pH-Werte zur Lagerung erhöht zu halten, z.B. bei pH-Wert 10, 11, 12 oder mehr, insbesondere pH 10 bis 13.

Aus Peroxochlorat kann sich über die Reaktion

$$2\ ^-OOClO_2 \rightarrow Cl_2O_6^{2-} + O_2$$

das Desoxo-Dimere bilden, bei dem die Chloratome in unterschiedlichen Oxidationsstufen (+ 3 und + 5) vorliegen. Diese Dimere sind ebenfalls neu und die zugehörige Säure und insbesondere ihre Salze und Anionen, wie auch ihre Herstellung und Verwendung, Gegenstand der Erfindung.

Peroxochtorat kann über die Reaktion mit Kohlendioxid bzw. deren Anionen Carbonat oder Hydrogencarbonat in ein Kohlendioxidaddukt überführt werden:

$$O_2ClOO^- + CO_2 \rightarrow O_2Cl\text{-}O\text{-}O\text{-}C(=O)O^-$$

und/oder

$$O_2ClOO^- + HCO_3^- \rightarrow O_2Cl\text{-}O\text{-}O\text{-}C(=O)O^- + OH^-$$

Diese Peroxoderivate mit Kohlensäure sind ebenfalls neu und sie, insbesondere ihre Salze und/oder Anionen, wie auch ihre Herstellung und Verwendung, sind Gegenstand der Erfindung (vgl. die Derivate von Peroxonitrit, ONOO$^-$, das unter ähnlichen Bedingungen ONOOC(=O)O bildet - Radi et al., Methods Enzymol. Vol 301, 353-67 (1999)).

Sofern in der vorliegenden Offenbarung von "Anionen" die Rede ist, ist die Gegenwart erforderlicher Gegenionen (vor allem in Lösung) eingeschlossen. Es soll mit der Bezeichnung "Anionen" vor allem zum Ausdruck gebracht werden, daß in Lösung das Peroxochiorat-Anion die stabilere Form gegenüber der protonierten Säure ist.

Das Peroxochlorat und das Desoxo-Dimer, wie auch das Kohlensäureaddukt, können erfindungsgemäß als Gemisch vorliegen.

Die Erfindung betrifft auch das Verfahren zur Herstellung von Peroxochlorsäure bzw. von deren Desoxo-Dimeren und/oder Kohlendioxiddäddukten und/oder Anionen und/oder Salzen dieser Verbindungen. Dieses Verfahren besteht darin Chlordioxid mit wäßrigen oder wasserhaltigem Wasserstoffperoxid bei einem pH-Wert von $\geq$ 10 bevorzugt pH 10 - 12 umzusetzen. Es ist bevorzugt, den pH-Wert auf einem konstanten Wert zu hatten.

Die Umsetzurig kann in wäßrigem Milieu oder in wasserhaltigem Milieu durchgeführt werden, Es können beispielsweise neben Wasser auch mit Wasser mischbare Lösungsmittel vorliegen, wie beispielsweise Alkohole, wie z.B. Alkanote, wie Methanol, Ethanol oder dergleichen, oder Mischungen davon.

Wahlweise kann auch von anderen Chloroxiden ausgegangen werden. So läßt sich beispielsweise Chlormonoxid vorzugsweise in seiner dimeren Form ($Cl_2O_2$) ebenfalls mit einem Hydroperoxid (vorzugsweise Wasserstoffperoxid) zum gewünschten Produkt umsetzen. Die Umsetzung gelingt im gleichen pH-Gebiet wie beim Chlordioxid angegeben.

Die Reaktionstemperatur kann erhöht werden, beispielsweise bis zu etwa 50°C; bei rein wäßrigen Systemen liegt die niedrigste Temperatur bevorzugt bei etwa 0°C. Liegen zusätzliche organische Lösungsmittel und/oder hohe Konzentrationen der beteiligten. Reagentien vor, so können auch niedrigere, d.h. unter dem Gefrierpunkt von Wasser liegende Temperaturen verwendet werden. Bevorzugt wird bei Raumtemperatur gearbeitet.

Das zur Umsetzung benötigte Chlordioxid steht dem Fachmann zur Verfügung und kann in üblicher Weise hergestellt werden. Beispielsweise kann es hergestellt werden durch Reaktion eines Chlorits mit einer Säure (beispielsweise Natriumchlorit mit Schwefelsäure) oder durch Reduktion von Chlorat, beispielsweise mit schwefeliger Säure.

Das so erhaltene Chlordioxid kann gegebenenfalls nach Entfernung von vorhandenen Spuren von Chlor in an sich bekannter Weise (Granstrom, Marvin L.; and Lee, G.Fred, J. Amer. Water Works Assoc. 50, 1453-1466 (1958)) verwendet werden.

Sollte das zur $ClO_2$-Herstellung eingesetzte Chlorit mit Carbonat verunreinigt sein, so entsteht $ClO_2$, das mit $CO_2$ verunreinigt ist, und/oder die oben beschriebenen Kohlensäureaddukte. Zur Absorption des Kohlendioxids sollte der Chlordioxid- und Kohlendioxid-hahige Gasstrom durch eine mit Lauge beschickte Waschflasche geleitet werden. Besser ist es jedoch, die Verunreinigungen an Carbonat durch fraktionierte Kristallisation des eingesetzten Natriumchlorit zu befreien. Eine Verunreinigung des Peroxochlorates-mit Carbonat läßt sich leicht im Ramanspektrum erkennen. Anstelle der schaden Bande bei 1051. cm$^{-1}$ erhält man eine Doppelbande bei 1069 cm$^{-1}$ (breit) und die im Rahmen der Erfindung wichtige Bande bei 1051 cm$^{-1}$ (scharf).

Das Chlordioxid kann mit einem Inertgas, wie Stickstoff oder einem, Edelgas, wie Argon, jedoch auch durch Luft oder Sauerstoff zur Reaktion mit dem Wasserstoffperoxid gefördert werden. Beispielsweise ist es möglich, das Chlordioxid in einem ersten Reaktionsgefäß herzustellen und mit den genannten Gasen oder einem Gemisch daraus in ein zweites Reaktionsgefäß einzuleiten, in dem sich Wasserstoffperoxid in wasserhaltiger oder wäßriger Lösung befindet.

Der pH-Wert des Reaktionsgemischs wird durch Zugabe einer Base gleich oder oberhalb 10 gehalten. Bevorzugt ist es, den pH-Wert konstant zu halten. Dies kann entweder manuell oder auch automatisch durch ein pA-Stat-Gerät erfolgen.

Als Basen können übliche anorganische, oder organische Basen, wie Alkalllaugen, beispielswiese Natronlauge oder Kalilauge oder Erdalkalihydroxide, Ammoniak oder organische Basen, wie Stickstoffbasen, dienen. Es können auch die Hydroxide von quaternären Ammoniumsalzen, insbesondere Alkyl-, wie Trialkylammoniumhydroxide, oder Zinkhydroxide eingesetzt werden.

Der Gehalt an Hydroperoxid in dem Reaktionsgemisch kann beispielsweise durch potentiometrische Titration mit einer Säure, wie beispielsweise Salzsäure, bestimmt werden.

Die nach dem vorstehend beschriebenen Verfahren erhaltenen Lösungen können als solche oder auch in abgewandelter Form zum Einsatz gebracht werden. Beispielsweise kann überschüssiges Wasserstoffperoxid in üblicher Weise, z.B. mit einer Schwermetallverbindung, wie Braunstein, beseitigt werden.

Es ist auch zweckmäßig, die Reaktion in Gegenwart von zusätzlichem Chlorit, beispielsweise Alkalimetallchlorit, z.B. Natriumchlorit, durchzuführen. Durch den Zusatz wird das Gleichgewicht der Zersetzungsreaktion

$$O_2ClOO^- \; {}_{<}{=}^{>} \; ClO_2^- + O_2$$

nach links verschoben, d. h. das Peroxochlorat stabilisiert.

Zur Verbesserung der Haltbarkeit des Reaktionsprodukts ist beispielsweise eine Lagerung bei erhöhtem pH-Wert geeignet, beispielsweise bei pH-Wert 10 oder mehr. Die Einstellung dieses pH-Werts kann mit einer geeigneten Base, wie vorstehend zum Herstellungsverfahren beschrieben, vorgenommen werden.

Zur Erzielung einer Chlorit-freien Peroxochlorsäure, ihrer Salze oder der Lösungen davon ist es überraschenderweise gelungen, die freie Säure bei Erniedrigung des pH-Wertes unter 6, z.B: auf pH-Wert 5 oder weniger aus dem erhaltenen Chlorationen - enthaltenden Gemisch mit einem Inertgas, wie einem Edelgas, z.B. Argon oder Stickstoff, auszutreiben und aufzufangen. Sie kann beispielsweise in einer Base, wie einer Alkalimetallbase, Erdalkalimetall- oder Zinkbase oder Stickstoffbase, wie Ammoniak oder einem organischen Amin aufgefangen werden. Es ist aber auch möglich die freie gasförmig anfallende Säure in einer Kühlfalle (z.B. bei -100 bis -190 °C) auszuftieren.

Beispielsweise ist es möglich, aus einer 2,7 Gew.-%-Lösung der Säure in der etwa 50-fachen Menge an Natriumchtorit die mit Argon ausgetriebene Säure zu 70 % mit 2M Natronlauge aufzufangen. Ein UV-Spektrum der Lösung zeigte durch das Fehlen des Peaks bei 260 nm, daß in der Lösung kein Natriumchlorit zugegen ist.

Einen weiteren Gegenstand der Erfindung bildet daher ein Verfahren zur Herstellung und Isolierung von Peroxochlorsäure und/oder deren Desoxo-Dimeren und/oder Kohlendioxidaddukten sowie Salzen davon, bei dem Chlordioxid mit einer wäßrigen oder wasserhaltigen Lösung von Wasserstoffperoxid bei einem pH-Wert von ≥ 10 bevorzugt 10-12 umgesetzt wird, worauf der pH-Wert unter 6 erniedrigt und die freie Säure oder deren Desoxo-Dimer als Gas mittels eines Inertgases aus der Lösung ausgetrieben und aufgefangen wird. Zur Gewinnung Kohlendioxidfreier Produkte wird hierbei vorzugsweise unter Luftabschluss, beispielsweise unter Stickstoff, Sauerstoff oder Argon, gearbeitet.

Wird die Säure in einer waßrigen Lösung einer Base aufgefangen, so erhält man die Chlorit-freien entsprechenden Salze in Lösung. Die Lösungen können in üblicher Weise eingeengt werden, z.B. durch Eindampfen unter reduziertem Druck, wobei man die Salze in kristalliner Form isolieren kann, oder durch Behandeln mit einem Gasstrom, wie Argon oder Stickstoff. Die Struktur kann durch eine Röntgenstrukturanalyse bestätigt werden. Als Gegenionen kommen alle Metallkationen und organische Kationen, wie diejenigen von Stickstoffbasen, insbesondere quaternäre Ammoniumsalte, in Frage. Welche Kationen besonders geeignet sind, ergibt sich aus dem jeweiligen Verwendungszweck. Für pharmazeutische Anwendungen sind insbesondere Erdalkali- oder Alkalimetalle, vozugsweise Na$^+$ oder K$^+$, oder Zn$^{2+}$ bevorzugt, bei technischen Anwendungen können auch organische Kationen, wie Kationen von Stickstoffbasen, insbesondere Alkylammoniumkationen, wie Trialkylammoniumkationen, oder vor allem quaternäre Ammoniumkationen eingesetzt werden.

Einen Hinweis auf das Vorliegen der Salze liefert auch die Wiederauffindbarkeit von Peroxochlorat-Anionen nach Wiederauflösung eines getrockneten Salzes durch Titration: Beispieisweise wird eine Lösung vom pH 10,5, die 0,1-molar an Natriumperoxochlorat ist (Gehalt durch Titration ermittelt), zur Trockene eingedampft und anschließend wieder in Wasser aufgenommen. Die Titration eines aliquoten Teils mit 0,1 M Salzsäure ergibt vorzugsweise eine Wiederfindung von 80% bis 100%, insbesondere 90 % bis 99,95%, beispielsweise 95 %.

Es ist zweckmäßig und bevorzugt, die Säure sowie die Salze unter Ausschuß von Licht zu lagern und daraus wäßrige Lösungen mit hohen pH-Werten herzustellen, z.B. mit pH-Werten von 10,11 oder 12 und darüber, insbesondere im Bereich von pH 10 bis pH 13, um lange Lagerfähigkeiten zu erzielen. Aus derartigen Lösungen kann je nach Bedarf die freie Säure, wie vorstehend beschrieben, wieder erhalten und gegebenenfalls in Lösungen mit gewünschtem pH-Wert bzw. in Salze überführt werden.

Die erfindungsgamäße Peroxochlorsäure oder ihre Desoxo-Dimere und/oder Kohlendioxidaddukte oder die Anionen oder insbesondere die Salze davon, liegen vorzugsweise in im wesentlichen reiner Form vor, d.h. in Lösung bezogen-auf die Gesamtmolarität der gelösten Peroxide insbesondere in mehr als 80 %, vorzugsweise mehr als 90 % Reinheit vor, vorzugsweise in mehr als 95 Mol-% Reinheit; im Fall der festen Salze in mehr als 80 Gewichtsprozent Reinheit, vorzugsweise in mehr als 90 Gewichts-% Reinheit, insbesondere in mehr als 95 Gewichts-% Reinheit.

Die erfindungsgemäße Peroxochlorsäure, deren Desoxo-Dimere und/oder Kohlendioxidaddukte bzw. Amionen und Salze dieser verbindungen können als solche und insbesondere in wäßriger oder wasserhaltiger Lösung als Oxidationsmittel für die verschiedensten medizinischen, kosmetischen, technischen und landwirtschaftlichen Zwecke Verwendung finden.

Beispiele für mögliche Testsysteme finden sich in den eingangs genannten Publikationen und Patentschriften/ die hier in dieser Hinsicht durch Bezugnahme aufgenommen werden.

Eine Anwendungsmöglichkeit besteht in der Verwendung als pharmazeutische Präparate (Arzneimittel) bzw. zur Herstellung von Arzneimitteln, die auf jegliche Weise, insbesondere topisch, aber auch parenteral verabreicht werden können. Die Arzneimittel können in üblicher Weise mit üblichen pharmazeutisch verträglichen Trägern und Verdünnungsmittein formuliert werden.

Die vorliegende Erfindung betrifft ebenfalls pharmazeutische Präparate, die als Wirkstoff Paroxoehljorsaure, Desoxo-Dimere und/oder Kohlendioxidaddukte und/oder Amionen und/oder Salze dieser Verbindungen, umfassen und insbesondere zur Behandlung der eingangs genannten Erkrankungen verwendet werden können. Besonders bevorzugt sind Präparate zur enteralen, wie nasalen; bukkalen, rektalen oder insbesondere oralen (vorzugsweise unter Umgehung der Magensäure, z.B. mit magensaftresistenten Präparaten wie Kapseln oder Lacktabletten, sowie vor allem zur lokalen oder parenteralen, wie intravenösen, intramuskulären oder subkutanen Verabreichung an Warmblüter, insbesondere Menschen. Die Präparate enthalten den Wirkstoff allein oder vorzugsweise zusammen mit einem oder mehreren pharmazeutisch anwendbaren Trägermaterialien. Die Dosierung des Wirkstoffs hängt von der zu behandelnden Krankheit, sowie von Spezies, deren Alter, Gewicht und individuellem Zustand, individuellen pharmakokinetischen Gegebenheiten sowie der Applikationsweise ab. Vorzugsweise liegt die Dosierung für die enterale oder insbesondere parenterale Applikation (beispielsweise durch Infusion oder Injektion) (vorzugsweise am Menschen) im Bereich von 0,01 bis 100 µMol/kg, insbesondere zwischen 0,1 bis 100 µMol, also beispielsweise bei einem Menschen mit einem Körpergewicht von 70 kg bei 1 mg bis 1 g/Tag, insbesondere bei 8,5 mg bis 850 mg/Tag, in einer Dosis oder aufgeteitt auf mehrere Dosen. Für die lokale Applikation liegen bevorzugte Dosisbereiche zwischen 0,1 und 10, insbesondere 0,5 und 5 ml/100 $cm^2$ einer 0,1 bis 10 millimolaren Lösung (entsprechend mehr oder weniger für grössere oder kleinere Flächen - applizierbar direkt oder unter Verwendung von getränkter Gaze).

So betrifft die Erfindung auch eine Verwendung der erfindungsgenaßen Verbindungen zur Herstellung eines Medikaments zur prophylaktischen und/oder therapeutischen Behandlung von hierin beschriebenen Krankheitszuständen, vorzugsweise zur prophylaktischen und/oder therapeutischen Behandlung von Krankheiten, bei deren Bekämpfung eine Verstärkung der Geweberegeneration, eine Immunmodulation, eine Verbesserung der Impfreaktion oder eine Strahlensensibilisierung angezeigt bzw. erfolgreich ist, oder zwei oder mehrere dieser Wirkungen, insbesondere zur Behandlung einer Wunderkrankung, bei einem Warmblüter, umfassend die Verabreichung von Peroxochlorsäure, deren Derivaten-und/oder Anionen, oder von Salzen davon, in einer gegen die genannten Krankheiten wirksamen Menge an einen Warmblüter, z.B. Menschen, der einer derartigen Behandlung bedarf.

Die Erfindung betrifft auch eine pharmazeutische Zusammensetzung zur prophylaktischen und insbesondere therapeutischen Behandlung von hierin beschriebenen Krankheitszuständen, vorzugsweise zur prophylaktischen oder therapeutischen Behandlung von Krankheiten, bei deren Bekämpfung eine Verstärkung der Geweberegeneration, eine Immunmodulation, eine Verbesserung der Impfreaktion oder eine Strahlensensibilisierung, angezeigt bzw. erfolgreich ist, oder zwei oder mehrere dieser Wirkungen, insbesondere einer Wunderkrankung, vorzugsweise eines Warmblüters, der an einer derartigen Erkrankung leidet, enthaltend Peroxochlorsäure, deren Derivate und/oder Anionen, oder Salze davon, in einer prophylaktisch oder insbesondere therapeutisch gegen die genannte Erkrankung wirksamen Menge und ein oder mehrere pharmazeutisch verwendbare Trägermaterialien.

Die Erfindung betrifft auch die Methode zur Verwendung bzw. ein Verfahren zur Verwendung von Peroxochlorsäure, deren Derivaten und/oder Anionen, oder Salzen davon, zur (kosmetischen) Pflege der Haut, beispielsweise bei Vorliegen einer Neigung zur Entwicklung von Pickeln oder Vorliegen von Pickeln.

Dosiseinheitsformen, sind z.B. Dragees, Tabletten, Ampullen, Vials, Suppositorien oder Kapseln. Weitere Applikationsformen insbesondere für Lösungen von Peroxochlorsäure, ihren Anionen und/oder Derivaten, oder Salzen davon, sind z.B. Salben, Crèmes, Pasten, Gele, Schäume, Mundwässer, Tropfen, Sprays, und dergleichen. Die Dosiseinheitsformen, z.B. Ampullen, Tabletten oder Kapseln, enthalten vorzugsweise von etwa 0,05 g bis etwa 1,0 g, insbesondere von 8,5 mg bis 850 mg, eines Salzes von Peroxochlorsäure und/oder deren Derivaten mit üblichen Trägermaterialien.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt.

In einer bevorzugten Ausführungsform kann eine 0,05 bis 1 M Lösung eines Peroxo-chloratsalzes und/oder eines Salzes von dessen Desoxo-Dimer und/oder Kohlendioxidaddukten in bidestilliertem Wasser bei einem pH gleich oder > 10 bis 13, insbesondere 12,5, gelöst werden.

Diese Lösung wird unmittelbar vor der Verabreichung mit Köchsälz, Natrium- oder Kaliumbicarbonat und bidestilliertem Wasser zur Isotonie auf Konzentrationen von ca. 1 - 5 mM verdünnt und dem physiologischen pH angenähert. Diese Lösung ist für die parenterale, bevorzugt intravenöse Anwendung geeignet.

Für eine bevoragte Formulierung eines Arzneimittels zur topischen Anwendung werden vorzugsweise Peroxocholrat und/oder dessen Desoxo-Dimer und/oder Kohlendioxiddukte als Salze in bidestilliertem Wasser mit Konzentrationen im unteren millimolaren bzw. oberen mikromolaren Bereich, vorzugsweise im Konzentrationsbereich von 0,5 - 5 mM mit pH gleich oder > 10, insbesondere 10 bis 13, bevorzugt z.B.11,5, gelöst und mit Glycerin, Kochsalz oder einem anderen geeigneten verträglichen, möglichst physiologischen Mittel auf Isotonie eingestellt. Weitere Zusätze sind möglich. Insbesondere sind bei Abfüllung des Arzneimittels in Plastikbehätter solche Zusatzstoffe geeignet, die Übergartgsmetaltspuren neutralisieren können, da Übergangsmetalle in der Wandung während Lagerung gelöst werden und eine Zersetzung des Wirkstoffes katalysieren können. Beispiele für solche Zusatzstoffe sind Oligo- oder Polyalkohole, wie Ethylenglykol, Desferrioxamin oder EDTA (z.B. als Dinatriumedeat). Die so erhaltene Lösung kann direkt auch auf Wunden verabreicht werden.

Das Anion der Peroxochlorsäure ist stabil, die Säure selber zerfällt relativ schnell. Eine Arzneiwirkstoffstabilisierung kann deswegen über den pH erreicht werden. Die Wirkstofflösung kann zur Verbesserung der Verträglichkeit unmittelbar vor Gebrauch durch eine Pufferverdünnung auf einen annähernd physiologischen Wert abgesenkt werden. Aus Gleichung 1 ergibt sich im Blut bei pH 7,4 eine chemische Halbwertszeit von ca. 2,4 Stunden. Dies reicht für die Entfaltung der pharmakologischen Wirkung im ganzen Körper aus, denn diese Wirkung beruht nicht auf der Rezeptor-Liganden-Interaktion eines konventionellen Pharmakons, sondern sie ist, wie bereits dargelegt, mit einer schnell und irreversibel ablaufenden Oxidationsreaktion verknüpft. Deren pharmakologische Wirkung hält an, solange die Zelle und/oder ihre chemisch veränderten Strukturen erhalten bleiben, ist also nicht nach Abdiffundieren einer Wirksubstanz von einem Rezeptor beendet.

Beispiele für Indikationsgebiete, bei denen eine Verstärkung der Geweberegeneration prophylaktisch oder insbesondere therapeutisch zur Behandlung einer Erkrankung erfolgreich ist, sind die Geweberegeneration nach physikalischen Schäden (wie z.B. stumpfe und scharfe Traumen, kurzweiliges Licht, radioaktive Strahlung) und nach chemischer Schädigung (z.B. durch Gewebegifte, wie Lost, Chemotherapeutika). Ein weiteres Anwendungsgebiet in diesem Bereich ist die Verbesserung der Wundheilung insbesondere von hartnäckigen, sogenannten "spontanen" Wunden, die aufgrund eines Grundleidens (beispielsweise Diabetes mellitus, Gefäßleiden, Immunsuppression oder altersbedingt) nicht heilen wollen. Zu derartigen Wunden gehören insbesondere Dekubitus und chronische Beinulcera. Unter Wundbehandlung ist hier insbesondere die Behandlung vofn Wunden auf der Haut, den Schleimhäuten und in Geweben, wie z.B. Leber, Herzmuskel oder Knochenmark, zu verstehen.

Ein weiteres Indikationsgebiet bezieht sich auf die prophylaktische oder insbesondere therapeutische Behandlung von Krankheiten bei denen eine Immunmodulation erfolgreich ist, besonders die Verbesserung der Immunabwehr, insbesondere nach schweren bakteriellen (bedingt sowohl durch aerobe als auch besonders durch anaerobe Keime) und parasitären und viralen Infekten. Besonders gilt das für solche Infekte, die eine Anergie hervorrufen, wie sie beispielsweise für mykobakterielle Infekte (Lepra und TBC) bekannt ist Auch eine Immunabwehrschwäche, wie sie beispielsweise bei Herpes- und HIV-Virus-Erkrankungen auftreten kann, ist ein potentielles Indikationsgebiet für eine Peroxochlbrathehandlung. Das durch eine pathologische Toleranz gelähmte Abwehrsystem wird durch die Therapie mit der von den Immunzellen als künstliches Gefahrensignal wahrgenommenen Peroxochtorsäure wieder in die Lage versetzt, physiotogisch zu reagieren. Eine solche immunnmodulierende Intervention ist keinesfalls gleichzusetzen mit einer Immunstimulation, die sich bei AIDS bisher nicht als ein erfolgversprechender Therapieansatz erwiesen hat. Auch im Frühstadium nach mutagenen, gentoxischen Traumen, wie sie beispielsweise durch eine Gammabestrahlung oder durch eine Vergiftung mit sogenannten chemischen Radiomimetika ausgelöst werden können, und/oder Zelltraumata, die zu einer malignen Zellentartung mit späterem bösartigen Tumorwachstum führen können, kann eine costimulierende Immunmodulation durch Peroxochlorat zur Anwendung kommen.

Ein weiteres Gebiet bezieht sich auf Krankheiten, bei deren Therapie oder insbesondere Prophylaxe eine Verbesserung der Impfreaktion angezeigt bzw. erfolgreich ist. Hier wirkt die erfindungsgemässe Peroxochlorsäure, deren Derivate, ihre Anionen oder Salze davon insbesondere als Adjuvans. Die Verbesserung einer schwachen Impfreaktion, wie sie insbesondere auch nach Verabreichung einer sogenannten Peptidovakzine bekannt ist, oder auch die oxidative Mitigierung einer Lebendvakzine gehören somit ebenfalls zu den Anwendungsgebieten.

Schließlich ist ein Indikationsgebiet die Strahlensensibilisierung, beispielsweise bei der Tumorbehandlung, nach oder vor der Bestrahlung oder simultan oder zeitlich überlappend.

Als Peroxoverbindung mit einer therapeutischen Breite von 2 log (i.p. $LD_{50}$ bei Ratten > 0,1 Millimol pro kg Körpergewicht) eignet sich Peroxochlorat insbesondere bei intratmoraler Anwendung zur Strahlensensibilierung, insbesondere in relativ kurzen Zeiträumen vor oder nach der Bestrahlung, bevorzugt 1 - 24 Stunden vor oder nach der Bestrahlung. Eine gewebeverträgliche biozide Desinfektion ist im Bereich $10^{-5}$ -$10^{-4}$ M (bevorzugter Konzentrationsbereich am Wirkort) möglich.

Peroxochlorsäure, die ein Analogen zum körpereigenen Wasserstoffperoxid darstellt, liegt beim pH-Wert des Blutes von 7,27 - 7,53 (Mittelwert 7,4) infolge des $pK_s$-Wertes von 6,5 ± 0,2 überwiegend, d.h. zu 89 Mol-%, als stabiles Anion und zu 11 Mol-% als Peroxosäure vor, während bei Wasserstoffperoxid ($pK_s$ =11,9) unter physiologischen Bedingungen der ungeladene metastabile $H_2O_2$ Anteil 99,997 Mol-% und das Hydroperoxidanion

(-OCH) lediglich 0,003 Mol-% beträgt, Insbesondere die ungeladene Säure kann daher in die Zellen eindringen, und da sie (anders als $H_2O_2$) gegen Katalase resistent ist, dort auch höhere Konzentrationen und damit mehr Wirksamkeit (z.B. bei der Regulation der Transkription oder Translation) erreichen als Wasserstoffperoxid.

Pharmakodynamisch sind aber in beiden Fällen im Körper unter anderem auch Produkte wirksam, die in vivo dadurch entstehen, daß nicht-ionisierte Peroxosäuren das Übergangsmetall Eisen in den Hämatoporphyrinen oxidieren. Oxidiertes Eisen (Fe>III) stellt als Fe(V)=0 bzw. Fe(IV)=0π* Porphyrine die terminale Wirkform dar. Als milde Oxidantien können die Fe(V)=Porphyrine in einem zweiten Schritt zu einer oxidativen Spaltung von Zuckerketten, insbesondere membranständiger Glykoproteine führen, die Regulatorfunktionen für Zellinteraktionen wahrnehmen (Rutishauser U., Acheson A., Hall A.K., Dennis M.M., Sunshine J.: "The Neural Adhesion Molecule as a Regulator of Cell-Cell Interactions" Science 240 : 53-57, 1988). Solche Glykoproteine mit Regulatortunktion spielen nach dem neuesten immunologischen Kenntnisstand auch bei der Interaktion antigenpräsentierender Zellen (APC) mit T-Lymphozyten eine entscheidende co-stimulierende Rolle (Austyn J.M. and Wood K.J. : 4,2 Membrane Molecules in T-cell responses in Principles of Cellular and Molecular Immunology, Oxford University Press, 1993).

Damit ist ein Hydroperoxid pharmakblogisch dann wirksam, wenn es auf Grund seiner Stabilität als Transportform (prodrug) angesehen werden kann. Für Peroxochlorat trifft das einerseits zu, weil es chemisch stabiler als das nicht-ionisierte $H_2O_2$ ist, andererseits wird es im Gegensatz zu dem letzteren nicht von dem Enzym Katalase angegriffen, die Wasserstoffperoxid zu Wasser und Sauerstoffgas abbaut Aus diesem Grunde kann Wasserstoffperoxid im Gegensatz zum Peroxochlorat nicht intravenös verabreicht werden. Als substituiertes Hydroperoxid (ROOH; R = $ClO_2$) hat Peroxochlorat einen wieteren biochemischen Vorteil gegenüber dem im Körper von Phagozyten gebildeten $H_2O_2$. Substituierte Hydroperoxide (ROOH; R ≠ H) wie.z.B. Benzoylperoxid, Peressigsäure, Percarbonsäure, Methyl- und Ethylperoxid) bilden die terminale pharmakologische Wirkform der Fe(V)=0 bzw. Fe(IV)=0 π* Porphyrine wesentlich schneller als $H_2O_2$. Ihre Geschwindigkeitskonstanten für eine solche Oxidationsreaktion von Hämoproteinen bei physiologischem pH sind groß (>> $10^6$ $M^{-1}$ $s^{-1}$). $H_2O_2$ bildet die terminale pharmakologische Wirkform der Fe(V)=0 bzw. Fe(IV)=0 π* Porphyrine nicht nur langsamer, es zerstört sie unter Freisetzung von molekularem Sauerstoff ($O_2$) auch wieder schneller reduktiv. Substituierte Hydroperoxide (Jones P., Mantle D., Davies D.M., Kelly H.C.: "Hydroperoxidase Activities of Ferrihemes. Heme Analogues of Peroxidase Enzyme Intermediates" Biochemistry 16, 18 : 3974 - 3978, 1977) sind schlechte Reduktoren der terminalen pharmakologischen Wirkform der Fe(V)=0 bzw. / Fe(IV)=0 π* Porphyrine (Frew J.E. and Jones P.: "Structure and Functional Properties of Peroxidases and Catalases" et ibid cit ref. in Advances in Inorganic and Bioorganic Mechanisms Vol. 3; edited by A.G. Sykes, Academic Press, 1984).

Pharmako-dynamisch wirkende (co)mitogene Oxidantien sind als solche als Arzneimittel insbesondere überall da einsetzbar, wo der Zustand des Immunsystems sich limitierend auf die Geweberegeneration auswirkt, gleich an welcher Stelle des Körpers sie zur Heilung und Genesung des Organismus stattfinden muß und gleich durch welche Faktoren eine Immunabwehrschwäche hervorgerufen wurde, ob durch bakterielle, virale oder parasitäre Infekte, ob durch eine intensive radioaktive Strahlenbelastung, ob durch andere physikalische Faktoren, ob durch eine massive zytotoxische Therapie, ob durch eine Stoffwechselerkrankung, wie z.B. Diabetes mellitus, oder Kombinationen hiervon. Die Peroxochlorsäureanionen sind als (co)mitogene Oxidantien selbstverständlich auch wirksame Adjuvantien zur Infektionsbekämpfung und zur Behebung einer Infektabwehrschwäche, wiederum unabhängig davon, wie diese Schwäche ausgelöst wurde, ob infektiös, physikalisch, chemotherapeutisch und/oder hormonell.

Das Peroxochlorsäureanion kann darüber hinaus in tierischen und pflanzlichen Geweben, die endoge Fe-porphyrine enthalten, als Desinfektionsmittel, besonders auch bei Anaerobier-Infektionen eingesetzt werden, sowie auch zur schonenden Desinfektion von zellulären und molekularen Blutbestandteilen.

Das Peroxochlorsäureanion kann beispielsweise auch als Desodorans in der Kosmetik (z.B. Achselhöhle, Füße) und in der medizinischen Pflege (beispielsweise Geschwüre), sowie aber auch in der Abwasserentsorgung verwendet werden, wenn es auf ein hohes Maß an Gewebe/Bioverträglichkeit ankommt.

In der Nahrungs- und Genußmittelindustrie kann das Peroxochlorsäureanion als hochwertiges, exzellentes Konservierungsmittel eingesetzt werden. Es hat sich gezeigt, daß Peroxochlorsäureanionen, in Spuren zu hochwertigen Rotweinen zugesetzt, diese sofort nach dem Entkorken ihr volles Bukett bei voller Erhaltung des Gaumen- und Zungengeschmacks entfalten lassen.

In der Technik, beispielsweise der chemischen Technik, kann das Peroxochlorsäureanion beispielsweise zur gezielten Oxidation von Doppelbindungen eingesetzt werden; beispielsweise zur Herstellung von Oxiranen oder entsprechenden Diolen. Beispielsweise ist es möglich, Propylen zu Oxiranen, wie Propylenoxid oder zu den entsprechenden Diolen zu oxidieren. Ein weiterer industrieller Anwendungszweck liegt in der Beseitigung von Geruchsbelästigungen, wie sie beispielsweise durch Kompostieranlagen, Räuchereien, Kraftwerke usw. hervorgerufen werden. Ein wichtiger Anwendungszweck ist die bereits erwähnte Entkeimung von beispielsweise Nahrungsmitteln wie Trinkwasser sowie der Einsatz auf dem Agrarbereich. Da es möglich ist, CC-Bindungen zu spalten, können die Peroxochlorsäure auch mit dem Ziel eingesetzt werden, Ballaststoffe aus Trink- oder Badewasser zu reduzieren bzw. diese Wässer zu entgiften.

Bei der vorstehend erwähnten Beseitigung der Geruchsbelästigung von beispielsweise Kraftwerken ist der erfindungsgemäße Einsatz auch von besonderer Bedeutung, da neben Nichtmetalloxiden wie Schwefeldioxid und Stickoxiden in der Abluft auch Schwermetalle enthalten sind. Diese werden zwar in der Regel von Filteranlagen zurückgehalten, dennoch stellt das Schwermetall Quecksilber ein Problem dar, da es garantiert sein muß, daß es vollständig in seiner zweiwertigen Form vorliegt. Dies kann durch den Einsatz der erfindungsgemäßen Peroxochlorsäure erzielt werden. Da es sich bei den erfindungsgemäßen Peroxochlorsäuren um definierte Verbindungen handelt, ergeben sich auch keine Schwierigkeiten bei der Arzneimittel-Zulassung.

Beispiel 1

Herstellung einer Metallperoxochloratlösung, die 50 bis 80 Gew.-% Metallchlorit enthalten kann und beispielsweise für technische Zwecke, wie z.B. Körperhygiene, zur Einleitung in Böden und Abwasser zur chemischen Dekontamination verwendet werden kann.

In einem ersten Reaktionsgefäß wird Chlordioxid durch Reaktion von Natriumchlorit und Schwefelsäure hergestellt. Das gasförmige Chlordioxid wird mit einem Stickstoffstrom ausgetrieben und in ein zweites Reaktionsgefäß eingeleitet, in dem sich ein halber Liter einer molaren Lösung von Wasserstoffperoxid in Wasser befindet. Bei einem ersten Versuch wird dieser Lösung kein Natriumchlorit, bei einem zweiten Versuch werden 0,1 M Natriumchlorit zugesetzt. Während des Einleitens des Gases wird der pH-Wert der Reaktionslösung mittels einer Einstab-Glaselektrode überwacht. Der pH-Wert wird durch automatische Zugabe mit einem pH-Stat-Gerät beim Neutralpunkt gehalten. Als Base wird Natronlauge verwendet. Der Fortschritt des Umsatzes an Chlordioxid wird über die zugegebene Menge der Natronlauge überwacht. Nach einem Verbrauch von 4 mmol Natronlauge enthalten die Lösungen beider Versuche 1 mmol des Anions des wirksamen Peroxochlorsäureanhydrids (Ausbeute 13 % d.Th.). Der Gehalt an Chlorhydroperoxid wird durch potentiometrische Titration mit Salzsäure bestimmt.

Zur Verbesserung der Haltbarkeit des Reaktionsprodukts wird der pH-Wert mit Natronlauge auf über 10 eingestellt.

Beispiel 2

Herstellung einer Metallperoxochloratfestsubstanz in einem Gemisch mit ca. 50 % Metallchlorit (für technische Zwecke, insbesondere zur Herstellung von Tabletten zur Aufbereitung keimfreien Trinkwassers)

Die Na-peroxochlorathaltige Lösung (hergestellt nach Beispiel 1) wird bis zur beginnenden Kristallisation eingedampft. Bei ca. +4°C scheiden sich die Kristalle von reinem Na-Chlorit ab. Diese werden abgesaugt. Die Mutterlauge wird zwei bis drei weiteren Kristallisationsschritten unterzogen, bis das ausfallende Na-Chlorit merklich Na-Peroxochlorathaltig ist. Der Gehalt von Peroxychlorat wird jeweils durch Titration mit 0,1 N HCl bestimmt.

Auf diese Weise kann eine etwa 1,5 molare Na-Peroxochloratlösung hergestellt werden, die zur Trockne eingedampft wird. Der dabei erhaltene farblose Feststoff enthält bis zu 60 % Natriumperoxochlorat, Natriumchlorit und in geringem Ausmaß Natriumchlorid sind als Verunreinigungen enthalten. Im Raman-Spektrum der so konzentrierten Lösung zeigen sich zwei Banden: Die Bande bei 799 cm$^{-1}$ ist charakteristisch für Chloritionen, die Bande bei 1051 cm$^{-1}$ ist Peroxochlorat zuzuordnen. Von Schritt zu Schritt der beschriebenen Anreicherung nimmt die Bande bei 1051 cm$^{-1}$ an Intensität zu, die Bande bei 799 cm$^{-1}$ ab. Das zum Natriumperoxochlorat isomere Natriumperchlorat zeigt im Raman-Spektrum eine Bande bei 938 cm$^{-1}$, für Natriumchlorat liegt die Bande bei 933 cm$^{-1}$.

Beispiel 3

Herstellung einer Peroxochloratlösung ohne Chloritverunreinigung (für pharmazeutische Zwecke)

Die Peroxochlorat-haltige Lösung wird mit einer Säure (z.B. Salzsäure, Schwefelsäure oder ähnlichem) auf einen pH-Wert um 5 eingestellt. Mit einem kräftigen Gasstrom (z.B. Argon) wird sofort die undissoziierte Säure ausgetrieben und in einer Waschflasche aufgefangen, die mit einer basischen Lösung beschickt ist (vorzugsweise eine 0,1 M NaOH). Schon nach wenigen Minuten ist die Peroxochlorsäure aus der Ausgangslösung vollständig ausgetrieben und zu ca. 70 % unzersetzt als stabiles Anion aufgefangen. Diese Auffanglösung enthält nur noch stabiles Metallperoxochlorat sowie nach der Rücktitration auf den physiologischen pH mit Säure (z.B. HCl oder CO$_2$) wenig Salz (z.B. NaCl oder NaHCO$_3$/lNa$_2$CO$_3$ neben Spuren von NaOH.

Beispiel 4

Herstellung einer Peroxochloratlösung ohne Chloritverunreinigungen

Eine möglichst konzentrierte Lösung eines Metall-, Ammonium- oder Alkylammoniumperoxochlorats, die Verunreinigungen an Chloritionen haben kann, wird zu einer Pufferlösung vorzugsweise vom pH zwischen 3,5 und 5,5 (beispielsweise Citronensäure- oder Phosphorsäure-Puffer) zugetropft. Der pH-Wert wird mit einer Glaselektrode überwacht. Er darf nicht höher als 6 sein. Mit einem kräftigen Gasstrom (beispielsweise Stickstoff, Sauerstoff, Argon oder auch $CO_2$-freie Luft) wird die Peroxochlorsäure ausgetrieben und in eine Batterie von drei hintereinandergeschalteten Waschflaschen geleitet, die mit einer Base beschickt sind. Es können beliebige Basen verwendet werden. Beispielsweise eignen sich Natronlauge oder Kalilauge, aber auch Ammoniaklösung und Lösungen von Erdalkali- oder Zinkhydroxiden sowie Stickstoffbasen-enthaltende Lösungen. Ebenso können Lösemittelgemische, wasserhaltige sowie wasserfreie organische Lösemittel verwendet werden. Es ist zu kontrollieren, daß der pH-Wert in den Waschflaschen möglichst nicht unter einen Wert von pH 10 abfällt.

Beispiel 5

Herstellung von reinem kristallinen Metall-, Ammonium- oder Alkylammoniumperoxochlorat

Die im Beispiel 3 oder 4 hergestellte chloritfreie Lösung an Metall-, Ammonium- oder Alkylammoniumperoxochlorat wird bis zur beginnenden Kristallisation durch Begasen mit Argon eingeengt. Im Falle von Natrium als Metallion scheiden sich farblose stäbchenförmige Kristalle an Natriumperoxochlorat ab. Die Kristalle werden abgesaugt, mit wenig Wasser gewaschen und an der Luft getrocknet.
Werden 5 mg des Natriumsalzes in 0,5 ml Wasser gelöst, so lässt sich im Ramanspektrum die charakteristische Bande bei 1051 cm$^{-1}$ als scharfer Peak erkennen. Die Titration mit 0,1 M Salzsäure ergibt einen Verbrauch von 0,4 ml.

Beispiel 6

Zu einer Lösung von 100 g Natriumchlorit in 200 ml Wasser gibt man unter Rühren vorsichtig tropfenweise Salzsäure (96%-ig) zu. Mit einem kräftigen Gasstrom ($N_2$ oder $O_2$) wird das entstehende Chlordioxid ausgetrieben. Der $ClO_2$-haltige Gasstrom wird über drei hintereinandergeschaltete Waschflaschen, die mit je 30 ml einer 2 M $NaClO_2$-Lösung vom pH 11 beschickt sind, in eine Lösung von 15 ml 30%-igem Wasserstoffperoxid in 35 ml Wasser, die zuvor durch Zugabe von 4M Natronlauge auf pH 12 gebracht ist, eingeleitet. Während der Gaseinleitung wird der pH-Wert mit einer Glaselektrode kontrolliert. Durch Zugabe von 4M NaOH wird der pH-Wert bei 12 gehalten. Das vorgelegte $H_2O_2$ ist verbraucht, wenn die Gaseinleitung zu einer Gelbfärbung führt. Mit einem Tropfen $H_2O_2$ wird die Lösung anschließend wieder entfärbt. Die peroxochlorathaltige Lösung wird unter Rühren zu einer Lösung von 500 g Zitronensäure in 3 Liter Wasser getropft, die zuvor mit 2 M Natronlauge auf ph 4,5 eingestellt ist. Während der Zugabe wird die Peroxochlorsäure mit einem kräftigen Gasstrom ($N_2$ oder $O_2$) ausgetrieben und in drei hintereinandergeschaltete Waschflaschen, die mit je 50 ml 0,1 M NaOH beschickt sind, aufgefangen. Wenn der pH-Wert in den Waschflaschen auf 10 gesunken ist, werden die Inhalte der Waschflaschen vereinigt und zur Trockene eingeengt. Ausbeute: 1,5 g trockenes Natriumperoxochlorat.

Beispiel 7

Anwendungsbeispiel

Bei 10 Patientfallbeispielen mit chronischen, infizierten und seit über 4 Monate bis zu einem Jahr therapierresistenten Wunden (2 diabetische Fußwunden, 4 Ulcera venosa, 1 Ulcus nach Laserung einer Fingerwarze, 1 Fingerwunde bei Scleroderma [links behandelt heilend, rechts trocken verbunden nicht heilend], 2 Wunden bei leukozytenklastischer Vaskulitis) kann eine Abheilung unter einer äußerlichen Wundfeuchtbehandlung mit 0,01 bis 0,001 %igem Peroxochlorat in physiologischer Kochsalzlösung erzielt werden. In diesen Fällen dauert bei dieser Anwendung von Natriumperoxochlorat "under compassionate use" der vollständige Wundverschluß nicht länger als zwei Monate.

**Patentansprüche**

**1.** Peroxochlorsäure, deren Desoxo-Dimere und/oder Kohlendioxidaddukte, und/oder Anionen und/oder Salze dieser Verbindungen.

2. Peroxochlorsäure, deren Desoxo-Dimere und/oder Kohlendioxidaddukte, und/oder Anionen und/oder Salze dieser Verbindungen nach Anspruch 1 in wasserhaltiger oder wäßriger Lösung.

3. Alkalimetall-, Erdalkalimetall-, Zink-, Ammonium- und Aminsalze von Peroxochlorsdure, deren Desoxo-Dimeren und/oder Kohlendioxidaddukten gemäß Anspruch 1.

4. Verfahren zur Herstellung und Isolierung von Peroxochlorsäure, deren Desoxo-Dimeren und/oder Kohlendioxidaddukten und/oder Anionen und/oder Salzen davon nach einem der Ansprüche 1 bis 3, **dadurch gekennzeichnet, daß** man Chlordioxid mit einer wäßrigen oder wasserhaltigen Lösung von Wasserstoffperoxid bei einem pH-Wert von $\geq 10$ umsetzt, den pH-Wert durch Zusatz einer Säure erniedrigt und die gasförmige freie Peroxochlorsäure oder deren oligomere Derivate und/oder Kohlendioxidaddukte mit einem Inertgas austreibt und auffängt.

5. Verfahren nach Anspruch 4, **dadurch gekennzeichnet, daß** man die freie Säure oder deren Desoxo-Dimere und/oder Kohlendioxidaddukte in einer Kühlfalle auffängt.

6. Verfahren nach Anspruch 4, **dadurch gekennzeichnet, daß** man die freie Säure und/oder deren Desoxo-Dimere und/oder deren Kohlendioxidaddukte in eine wäßrige alkalische Lösung einleitet.

7. Verfahren nach Anspruch 6, **dadurch gekennzeichnet, daß** man als Base eine Alkalimetall-, Erdalkalimetall-, Zink- oder Stickstoffbase oder ein Hydroxid eines quaternären Ammoniumsalzes verwendet.

8. Verfahren nach Anspruch 7, **dadurch gekennzeichnet, daß** man die erhaltenen Salze durch Einengen oder fraktioniertes Kristallisieren isoliert.

9. Verfahren nach Anspruch 6 oder7, **dadurch gekennzeichnet, daß** man die erhaltenen Lösungen durch Erhöhen des pH-Wertes stabilisiert.

10. Pharmazeutisches Präparat, enthaltend Peroxochlorsaure, deren Desoxo-Dimere und/oder Kohlendioxidaddukte, und/oder Anionen und/oder Salze Verbindungen nach einem der Ansprüche 1 bis 3.

11. Pharmazeutisches Präparat nach Anspruch 10, **dadurch gekennzeichnet, daß** es zur parenteralen oder topischen Verabreichung formuliert ist.

12. Verwendung der Peroxochlorsäure, von Desoxo-Dimeren und/oder Kohlendioxidaddukten, und/oder Anionen und/oder Salzen dieser Verbindungen nach einem der Ansprüche 1 bis 3 als Oxidationsmittel, Desinfektionsmittel, Konservierungsmittel und/oder Bleichmittel.

13. Peroxochlorsäure, Desoxo-Dimere und/oder Kohlendioxidaddukte, und/oder Anionen und/oder Salze dieser Verbindungen gemäß einem der Ansprüche 1 bis 3 zur Verwendung als Medikament.

14. Verwendung von Peroxochlorsäure, deren Desoxo-Dimeren und/oder Kohlendioxidaddukten, und/oder Anionen und/oder Salzen dieser Verbindungen gemäß einem der Ansprüche 1 bis 3 zur Herstellung eines pharmazeutischen Präparates zur Behandlung des menschlichen oder tierischen Körpers, vorzugsweise zur prophylaktischen und/oder therapeutischen Behandlung insbesondere bei einem Warmblüter, insbesondere einem Menschen von Krankheiten, bei deren Bekämpfung eine Verstärkung der Geweberegeneration, eine Immunmodulation, eine Verbesserung der Impfreaktion und/oder eine Strahlensensibilisierung angezeigt bzw. erfolgreich ist, insbesondere zur Wundbehandlung.

15. Pharmazeutische Zusammensetzung zur prophylaktischen und insbesondere therapeutischen Behandlung von Erkrankungen, vorzugsweise zur prophylaktischen und/oder therapeutischen Behandlung - insbesondere bei einem Warmblüter, insbesondere einem Menschen - von Krankheiten, bei deren Bekämpfung eine Verstärkung der Geweberegeneration, eine Immunmodulation, eine Verbesserung der Impfreaktion oder eine Strahlensensibilisierung angezeigt bzw. erfolgreich ist, insbesondere einer Wunderkrankung, eines Warmblüters, der an einer derartigen Erkrankung leidet, enthaltend Peroxochlorsäure, deren Desoxo-Dimere und/oder Kohlendioxidaddukte, und/oder Anionen und/oder Salze dieser Verbindungen gemäss Anspruch 1 in einer prophylaktisch oder insbesondere therapeutisch gegen die genannte Erkrankung wirksamen Menge und ein oder mehrere pharmazeutisch verwendbare Trägermaterialien.

**Claims**

1. Peroxochloric acid, its desoxo-dimers and/or carbon dioxide adducts, and/or anions and/or salts of those compounds.

2. Peroxochloric acid, its desoxo-dimers and/or carbon dioxide adducts, and/or anions and/or salts of those compounds according to claim 1, in water-containing or aqueous solution.

3. Alkali metal, alkaline earth metal, zinc, ammonium and amine salts of peroxochloric acid, its desoxo-dimers and/or carbon dioxide adducts according to claim 1.

4. Process for the preparation and isolation of peroxochloric acid, its desoxo-dimers and/or carbon dioxide adducts, and/or anions and/or salts thereof according to any one of claims 1 to 3, **characterised in that** chlorine dioxide is reacted with an aqueous or water-containing solution of hydrogen peroxide at a pH value of $\geq 10$, the pH value is reduced by addition of an acid and the gaseous free peroxochloric acid or its oligomeric derivatives and/or carbon dioxide adducts are driven out using an inert gas and collected.

5. Process according to claim 4, **characterised in that** the free acid or its desoxo-dimers and/or carbon dioxide adducts are collected in a cold trap.

6. Process according to claim 4, **characterised in that** the free acid and/or its desoxo-dimers and/or its carbon dioxide adducts are introduced into an aqueous alkaline solution.

7. Process according to claim 6, **characterised in that** there is used as base an alkali metal, alkaline earth metal, zinc or nitrogen base or a hydroxide of a quaternary ammonium salt.

8. Process according to claim 7, **characterised in that** the salts obtained are isolated by concentration or by fractional crystallisation.

9. Process according to claim 6 or 7, **characterised in that** the solutions obtained are stabilised by increasing the pH value.

10. Pharmaceutical preparation comprising peroxochloric acid, its desoxo-dimers and/or carbon dioxide adducts, and/or anions and/or salts of those compounds according to any one of claims 1 to 3.

11. Pharmaceutical preparation according to claim 10, **characterised in that** it is formulated for parenteral or topical administration.

12. Use of peroxochloric acid, of desoxo-dimers and/or carbon dioxide adducts, and/or anions and/or salts of those compounds according to any one of claims 1 to 3 as oxidising agents, disinfectants, preservatives and/or bleaching agents.

13. Peroxochloric acid, desoxo-dimers and/or carbon dioxide adducts, and/or anions and/or salts of those compounds according to any one of claims 1 to 3 for use as medicaments.

14. Use of peroxochloric acid, its desoxo-dimers and/or carbon dioxide adducts, and/or anions and/or salts of those compounds according to any one of claims 1 to 3 in the manufacture of a pharmaceutical preparation for the treatment of the human or animal body, preferably for the prophylactic and/or therapeutic treatment - especially in a mammal, especially a human - of diseases in the control of which an enhancement of tissue regeneration, immunomodulation, improvement of vaccination reaction and/or a radiosensitisation is indicated or successful, especially for the treatment of wounds.

15. Pharmaceutical composition for the prophylactic and especially therapeutic treatment of diseases, preferably for the prophylactic and/or therapeutic treatment - especially in a mammal, especially a human - of diseases in the control of which an enhancement of tissue regeneration, immunomodulation, improvement of vaccination reaction and/or a radiosensitisation is indicated or successful, especially a wound disease, in a mammal suffering from such a disease, comprising peroxochloric acid, its desoxo-dimers and/or carbon dioxide adducts, and/or anions and/or salts of those compounds according to claim 1 in an amount that is prophylactically or, especially, therapeutically effective against the said disease, and comprising one or more pharmaceutically acceptable carrier materials.

**Revendications**

1. Acide peroxochlorique, ses dimères désoxo et/ou adduits de dioxyde de carbone et/ou anions et/ou sels de ces composés.

2. Acide peroxochlorique, ses dimères désoxo et/ou adduits de dioxyde de carbone et/ou anions et/ou sels de ces composés selon la revendication 1, en solution hydratée ou aqueuse.

3. Sels de métaux alcalins, alcalinoterreux, de zinc, d'ammonium et amine d'acide peroxochlorique, de ses dimères désoxo et/ou adduits de dioxyde de carbone selon la revendication 1.

4. Procédé de préparation et d'isolement de l'acide peroxochlorique, de ses dimères désoxo et/ou adduits de dioxyde de carbone et/ou anions et/ou sels de ceux-ci selon l'une des revendications 1 à 3, **caractérisé en ce que** l'on fait réagir du dioxyde de chlore avec une solution aqueuse ou contenant de l'eau de peroxyde d'hydrogène à un pH $\geq 10$, que l'on diminue le pH par l'ajout d'un acide et que l'on extrait et capte avec un gaz inerte l'acide peroxochlorique libre gazeux ou ses dérivés oligomères et/ou adduits de dioxyde de carbone.

5. Procédé selon la revendication 4, **caractérisé en ce que** l'on capte dans un piège cryogénique les acides libres ou leurs dimères déxoxo et/ou adduits de dioxyde de carbone.

6. Procédé selon la revendication 4, **caractérisé en ce que** l'on fait passer dans une solution aqueuse alcaline les acides libres et/ou leurs dimères désoxo et/ou leurs adduits de dioxyde de carbone.

7. Procédé selon la revendication 6, **caractérisé en ce que** l'on emploie comme base une base de métal alcalin, alcalinoterreux, de zinc ou d'azote, ou un hydroxyde d'un sel d'ammonium quaternaire.

8. Procédé selon la revendication 7, **caractérisé en ce que** l'on isole les sels obtenus par concentration ou cristallisation fractionnée.

9. Procédé selon la revendication 6 ou 7, **caractérisé en ce que** l'on stabilise les solutions obtenues par augmentation du pH.

10. Préparation pharmaceutique contenant de l'acide peroxochlorique, ses dimères désoxo et/ou adduits de dioxyde de carbone et/ou anions et/ou sels de ces composés selon l'une des revendications 1 à 3.

11. Préparation pharmaceutique selon la revendication 10, **caractérisée en ce qu'**elle est formulée pour une administration parentérale ou topique.

12. Utilisation de l'acide peroxochlorique, ses dimères désoxo et/ou adduits de dioxyde de carbone et/ou anions et/ou sels de ces composés selon l'une des revendications 1 à 3, comme oxydant, désinfectant, conservateur et/ou décolorant.

13. Acide peroxochlorique, dimères désoxo et/ou adduits de dioxyde de carbone et/ou anions et/ou sels de ces composés selon l'une des revendications 1 à 3, pour une utilisation comme médicament.

14. Utilisation d'acide peroxochlorique, de ses dimères désoxo et/ou adduits de dioxyde de carbone et/ou anions et/ou des sels de ces composés selon l'une des revendications 1 à 3, pour la production d'une préparation pharmaceutique pour le traitement du corps humain ou animal, de préférence pour le traitement prophylactique et/ou thérapeutique, en particulier chez un animal à sang chaud, en particulier chez l'homme, de maladies dans le traitement desquelles un renforcement de la régénération des tissus, une modulation immunitaire, une amélioration de la réaction au vaccin et/ou une sensibilisation au rayonnement est indiquée ou est un succès, notamment pour le traitement des plaies.

15. Composition pharmaceutique pour le traitement prophylactique et notamment thérapeutique de maladies, de préférence pour le traitement prophylactique et/ou thérapeutique d'un animal à sang chaud notamment, en particulier d'un homme, de maladies dans le traitement desquelles un renforcement de la régénération des tissus, une modulation immunitaire, une amélioration de la réaction au vaccin et/ou une sensibilisation au rayonnement est indiquée ou est un succès, notamment d'une maladie avec suppuration d'un animal à sang chaud souffrant d'une maladie de ce type, contenant de l'acide peroxochlorique, ses dimères désoxo et/ou adduits de dioxyde de carbone et/ou anions et/ou des sels de ces composés selon la revendication 1, dans une quantité prophylactique, ou thérapeutique notamment, efficace contre la maladie citée, et un ou plusieurs supports utilisables pharmaceutiquement.

Fig. 1